# EUROPEAN PATENT APPLICATION

(11) **EP 4 741 002 A1**
(43) Date of publication of application: **13.05.2026**
(21) Application number: 24843338.5
(22) Date of filing: 21.06.2024
(51) Int. Cl.: A61N 1/40, A61N 1/32, A61N 1/36, A61N 1/08, A61N 1/06

(54) **SKIN TREATMENT DEVICE AND DRIVING METHOD THEREOF**

(30) Priority: 17.07.2023 KR 20230092489
(71) Applicant: Piamolifting.Co.,Ltd, Gyeongsangbuk-do 38577 (KR)
(72) Inventor: PARK, Won Hee, Seoul 06715 (KR)
(74) Representative: Flügel Preissner Schober Seidel
(86) International application number: PCT/KR2024/008636
(87) International publication number: WO 2025/018618

(57) **Abstract**

A skin treatment apparatus and a driving method thereof are disclosed. A skin treatment apparatus according to an embodiment of the present invention includes: a skin accommodating cap having an open lower surface and a cavity therein; an electrode pair provided such that, when a target portion of skin is suctioned toward the cavity of the skin accommodating cap, the suctioned skin comes into contact within the cavity of the skin accommodating cap, the electrode pair being disposed to face each other in a first direction; a driving unit configured to drive a pressing plate so that the pressing plate can advance into and retreat from an inner direction of the cavity; a controller for controlling the driving unit; and at least one pressing plate which is driven by the driving unit so as to be able to advance into the cavity from a second direction different from the first direction and which increases an area of contact with the electrode pair by pressing inward the skin suctioned into the cavity.

## Description

### [TECHNICAL FIELD]

The present invention relates to a skin treatment apparatus and a driving method thereof. In particular, the present invention relates to a technical concept capable of improving treatment efficiency in a method of treating skin by irradiating the skin with electrical energy.

### [BACKGROUND ART]

A method of performing skin disease treatment and fat treatment using electrical energy such as RF pulse energy or high-frequency energy is known.

As an example, there are a unipolar method in which the body is used as a (-) ground and a (+) handpiece is brought into contact with the skin and used in a vertical descending manner, and a bipolar method in which the skin is suctioned and electrical energy is applied from both sides.

One example of such a bipolar method is also disclosed in a Korean registered patent filed and registered by the present applicant (Registration No. 10-2407711, "Therapeutic Handpiece Having Vacuum Cap", hereinafter referred to as the 'Prior Patent'). The contents described in the Prior Patent may be incorporated as a reference of the present invention. As disclosed in the Prior Patent, when the skin is suctioned to form the skin threedimensionally and electrical energy is irradiated through disposed electrodes, fatty components can be melted to obtain a therapeutic effect.

However, such a conventional method has a problem in that the skin may not adhere well to the electrodes.

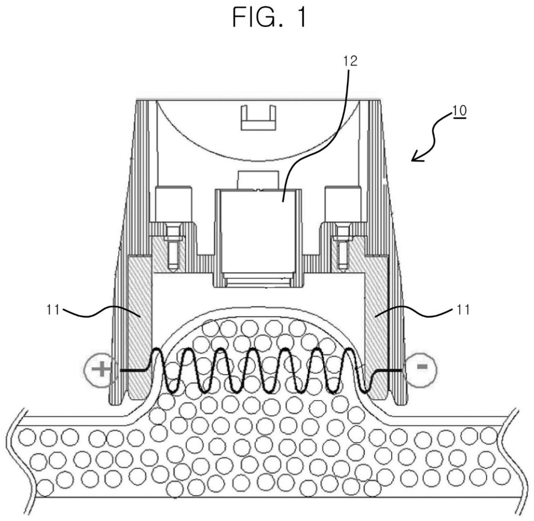

FIG. 1 is a view for explaining a problem of a conventional skin treatment apparatus.

As shown in FIG. 1, a conventional skin treatment apparatus 10 forms a negative pressure through a through-hole 12 to suction the skin into a cavity provided in the skin treatment apparatus 10, and then performs skin treatment by irradiating electrical energy through an electrode pair 11.

However, as shown in FIG. 1, when the skin is suctioned, the suctioned skin is formed like a mountain peak, so the degree of close contact with the electrodes disposed at both ends becomes significantly low. In general, when suction is performed by simply applying negative pressure through a vacuum device, only about 30 to 40% of the suctioned skin area comes into contact with the electrodes, and the remaining 60 to 70% does not adhere. Since electrical energy is irradiated only to the adhered skin region, there is a problem that the effect of skin treatment is reduced. That is, even if the suction pressure of the skin is increased, there is a limit to the degree to which the skin adheres to the electrodes.

### * Prior Art Documents

### - Patent Documents

(Patent Document 0001) Patent Document 1. Korean Registered Patent (Registration No. 10-2407711, "Therapeutic Handpiece Having Vacuum Cap")

### [DETAILED DESCRIPTION OF THE INVENTION]

### [TECHNICAL PROBLEM]

An object to be solved by the present invention is to provide a technical concept capable of dramatically increasing an area of contact with electrodes in a state where the skin is suctioned into a cavity, thereby improving a therapeutic effect through electrical energy. Another object is to provide a technical concept capable of applying pressure to the skin to provide a massage effect, thereby causing the tissue of skin fat to be destroyed or softened so that it can be more readily decomposed by electrical energy.

### [SOLUTION TO PROBLEM]

A skin treatment apparatus according to an embodiment of the present invention for solving the above technical problem includes: a skin accommodating cap having an open lower surface and a cavity therein; an electrode pair provided such that, when a target portion of skin is suctioned toward the cavity of the skin accommodating cap, the suctioned skin comes into contact within the cavity of the skin accommodating cap, the electrode pair being disposed to face each other in a first direction; a driving unit configured to drive a pressing plate so that the pressing plate can advance into and retreat from an inner direction of the cavity; a controller for controlling the driving unit; and at least one pressing plate which is driven by the driving unit so as to be able to advance into the cavity from a second direction different from the first direction and which increases an area of contact with the electrode pair by pressing inward the skin suctioned into the cavity.

The skin treatment apparatus may include a pair of pressing plates disposed to face each other in the second direction.

The controller may control the electrode pair to differentially perform whether to irradiate an RF pulse or an irradiation intensity according to driving of the pressing plate.

At least one of the at least one pressing plate or the electrode pair may be formed to be inclined at a predetermined angle such that a lower portion of an inner surface facing the inside of the cavity protrudes inward more than an upper portion thereof.

The driving unit may further drive the electrode pair so that the electrode pair can advance into and retreat from the inner direction of the cavity.

The skin treatment apparatus may further include a cooling module capable of cooling the electrode pair.

The skin treatment apparatus may further include a vacuum pump that provides negative pressure so that the target portion of the skin can be suctioned toward the cavity of the skin accommodating cap.

The driving unit may repeatedly advance and retreat at least one of the pressing plate or the electrode pair at a predetermined frequency.

The skin treatment apparatus may further include a temperature sensor for sensing a temperature of the skin suctioned into the cavity, and the controller may control the driving unit or control operation of the skin treatment apparatus based on the sensed temperature.

In another aspect, a skin treatment apparatus includes: a skin accommodating cap having an open lower surface and a cavity therein; an electrode pair provided such that, when a target portion of skin is suctioned toward the cavity of the skin accommodating cap, the suctioned skin comes into contact within the cavity of the skin accommodating cap, the electrode pair being disposed to face each other in a first direction; a driving unit configured to drive the electrode pair so that the electrode pair can advance into and retreat from an inner direction of the cavity; and a controller for controlling the driving unit, wherein the electrode pair is driven by the driving unit to press inward the skin suctioned into the cavity, thereby increasing an area of contact with the electrode pair.

A driving method of the skin treatment apparatus according to the technical concept of the present invention includes: suctioning a target portion of skin toward a cavity of a skin accommodating cap by the skin treatment apparatus; advancing a pressing plate into the cavity from a second direction different from a first direction of an electrode pair disposed in the first direction so that the suctioned skin comes into contact with the electrode pair within the cavity of the skin accommodating cap; and irradiating electrical energy through the electrode pair by the skin treatment apparatus.

The driving method of the skin treatment apparatus may further include repeatedly advancing and retreating at least one of the pressing plate or the electrode pair at a predetermined frequency by the skin treatment apparatus.

In another aspect, a driving method of a skin treatment apparatus includes: suctioning a target portion of skin toward a cavity of a skin accommodating cap by the skin treatment apparatus; advancing the electrode pair disposed in a first direction into an inner side of the cavity so that the suctioned skin comes into contact with the electrode pair within the cavity of the skin accommodating cap; and irradiating electrical energy through the electrode pair by the skin treatment apparatus.

### [ADVANTAGEOUS EFFECTS OF INVENTION]

According to an embodiment of the present invention, while the skin is suctioned into the cavity, the skin is pushed toward the center of the cavity by a pressing plate and/or electrodes, thereby dramatically increasing an area of contact with the electrodes, increasing a skin region to which electrical energy is applied, and thereby enhancing a therapeutic effect.

In addition, by applying pressure to the skin to provide a massage effect, the tissue of skin fat is destroyed or softened so that it can be more readily melted/decomposed by electrical energy.

### [BRIEF DESCRIPTION OF THE DRAWINGS]

In order to more fully understand the drawings cited in the detailed description of the present invention, brief descriptions of the respective drawings are provided.
FIG. 1 is a view for explaining a problem of a conventional skin treatment apparatus.
FIG. 2 is a view illustrating a schematic configuration of a skin treatment apparatus according to an embodiment of the present invention.
FIG. 3 is a view for explaining a skin accommodating cap of a skin treatment apparatus according to an embodiment of the present invention.
FIG. 4 is a view for explaining the inside of a skin accommodating cap when a pressing plate has advanced according to an embodiment of the present invention.
FIGS. 5 to 7 are views for explaining effects according to a driving method of the skin treatment apparatus according to an embodiment of the present invention.
FIG. 8 is a view illustrating a schematic flow chart for explaining a driving method of the skin treatment apparatus according to an embodiment of the present invention.

### [BEST MODE FOR CARRYING OUT THE INVENTION]

The present invention may be modified in various ways and may have various embodiments, and specific embodiments will be illustrated in the drawings and described in detail. However, this is not intended to limit the present invention to specific embodiments, and it should be understood to include all modifications, equivalents, and substitutes included in the spirit and technical scope of the present invention. In describing the present invention, if it is determined that a detailed description of related known technologies may obscure the gist of the present invention, the detailed description thereof will be omitted.

Terms such as first and second may be used to describe various components, but the components should not be limited by such terms. Such terms are used only for the purpose of distinguishing one component from another component.

Terms used in the present application are merely used to describe specific embodiments and are not intended to limit the present invention. Singular expressions include plural expressions unless the context clearly indicates otherwise.

In this specification, terms such as "include" or "have" are intended to specify that features, numbers, steps, operations, components, parts, or combinations thereof described in the specification exist, and should not be understood as precluding in advance the existence or addition of one or more other features, numbers, steps, operations, components, parts, or combinations thereof.

Hereinafter, the present invention will be described in detail with reference to embodiments of the present invention with reference to the accompanying drawings. The same reference numerals presented in the respective drawings denote the same members.

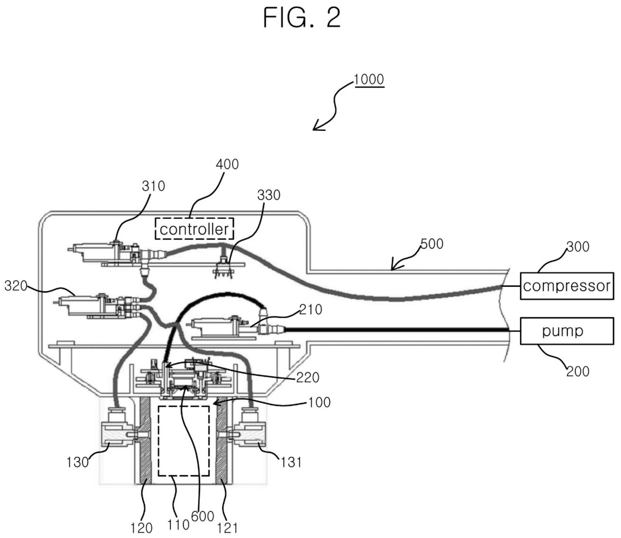

FIG. 2 is a view illustrating a schematic configuration of a skin treatment apparatus according to an embodiment of the present invention.

Referring to FIG. 2, a skin treatment apparatus 1000 according to the technical concept of the present invention may include a skin accommodating cap 100. The skin accommodating cap 100 includes a space into which the skin can be suctioned, that is, a cavity, as disclosed in the Prior Patent (a vacuum cap in the Prior Patent).

According to an embodiment, the cavity may have a quadrangular prism shape having a rectangular cross-section, but is not necessarily limited thereto. The cavity has an open lower surface, and the skin may be suctioned through the open lower surface.

The skin accommodating cap 100 may be provided with an electrode pair 110 disposed to face each other in a first direction (for example, a depth direction of the drawing) so that, when a target portion of the skin is attracted toward the cavity of the skin accommodating cap 100, the attracted skin may contact with the electrode pair within the cavity of the skin accommodating cap 100. Electrical energy may be applied to the skin in contact with the electrode pair 110 through the electrode pair 110. The electrode pair 110 may be implemented to irradiate electrical energy in various manners and embodiments, such as an RF pulse, high-frequency current, and the like.

In addition, the skin accommodating cap 100 may be provided, according to the technical concept of the present invention, with at least one pressing plate 120, 121 capable of advancing toward and retreating from the central direction of the cavity. The pressing plates 120, 121 may be disposed to face each other in a second direction (for example, a left-right direction of the drawing) different from the first direction (for example, the depth direction of the drawing). When the pressing plates 120, 121 advance toward the center through the driving units 130, 131, the skin sucked into the cavity is pushed toward the center, and in this case, the volume of the cavity is reduced so that the area of skin contacting the electrode pair 110 can be greatly increased.

Accordingly, the technical concept of the present invention can increase the contact area between the target portion of the suctioned skin and the electrode pair 110 by suctioning the skin into the cavity and then mechanically pushing the skin toward the center using the pressing plates 120, 121. Therefore, the region of skin to which electrical energy irradiated through the electrode pair 110 is applied can be enlarged, and thus a fat decomposition effect can be increased.

The skin accommodating cap 100 may be coupled with a body portion 500 to implement the technical concept of the present invention.

As disclosed in the Prior Patent, the body portion 500 may be rotatably coupled to the skin accommodating cap 100, thereby allowing a practitioner to easily adjust an angle to a desired angle during skin treatment while holding the body portion 500.

In order to implement the technical concept of the present invention, the skin needs to be suctioned into the cavity, and various methods may be possible.

According to one embodiment, the skin treatment apparatus 1000 may suction the skin by applying negative pressure to the cavity through a vacuum pump (or vacuum device, 200). The vacuum pump 200 is connected through a vacuum valve 210 and predetermined piping, and the vacuum valve 210 may be connected to a through-hole 220 communicating with the skin accommodating cap 100. Thus, a predetermined negative pressure may be applied to the skin accommodating cap 100. A predetermined vacuum pressure sensor (not shown) may be provided in the skin accommodating cap 100 or the vacuum valve 210, and thus the negative pressure applied to the cavity may be sensed.

The negative pressure applied to the cavity may be controlled by the controller 400. The controller 400 may control the vacuum pump 200 and/or the vacuum valve 210 to control the negative pressure applied to the cavity. According to one embodiment, the controller 400 may provide physical stimulation in which the skin is suctioned into the cavity and moves upward and downward while changing the magnitude of the negative pressure applied to the cavity at a predetermined period, thereby providing not only a massage effect but also an effect of destroying or softly massaging fat tissue inside the skin.

Although the present specification illustratively describes a case in which the skin is suctioned into the cavity by providing negative pressure through the vacuum pump 200, the present invention is not necessarily limited thereto. For example, a target portion of skin may be made to protrude upward by pinching the skin by hand, and then the skin may be manually inserted into the cavity.

Meanwhile, the skin accommodating cap 100 of the skin treatment apparatus 1000 may be provided with driving units 130, 131.

The driving units 130, 131 may drive at least one pressing plate 120, 121 according to the technical concept of the present invention. FIG. 2 illustrates an example in which the pressing plates 120, 121 are provided to face each other toward the center of the cavity, but the present invention is not necessarily limited thereto. For example, only one of the pressing plates 120, 121 may be provided and the other may be fixedly disposed. Even in such a case, those skilled in the art would readily infer that the area of skin contacting the electrode pair 110 can be increased compared to the conventional case in which the pressing plates 120, 121 are not provided.

Meanwhile, although the present specification mainly describes a case in which the pressing plates 120, 121 advance toward and retreat from the center direction of the cavity by the driving units 130, 131, in some embodiments, the skin treatment apparatus 1000 may be implemented such that the electrode pair 110 may also advance toward and retreat from the center direction of the cavity.

That is, instead of providing separate pressing plates 120, 121 in the skin accommodating cap 100, the electrode pair 110 may be implemented to advance toward and retreat from the center direction of the cavity, and in such a case, the electrode pair 110 may be implemented to be connected to a predetermined driving unit.

According to another embodiment, a predetermined driving unit (for example, a cylinder) may be connected to both the electrode pair 110 and the pressing plates 120, 121, and in this case, both the electrode pair 110 and the pressing plates 120, 121 may be able to advance into and retreat from the center of the cavity.

Various embodiments are possible. In the present specification, a case in which a pair of pressing plates 120, 121 is disposed to face each other and can advance into and retreat from the center of the cavity is mainly described, but the scope of the present invention is not limited thereto.

The driving units 130, 131 may be implemented as cylinders capable of advancing or retreating the pressing plates 120, 121. FIG. 2 illustrates an embodiment in which the driving units 130, 131 are implemented as air cylinders, but those skilled in the art would readily infer that the driving units 130, 131 may be implemented in various ways, such as oil cylinders, solenoids, and the like.

When the driving units 130, 131 are implemented as air cylinders, the driving units 130, 131 may advance or retreat through pressure of gas provided by a compressor 300. The compressor 300 can adjust pressure by compressing or expanding a predetermined gas, and the compressor 300 may be connected to the driving units 130, 131 through at least one valve (for example, solenoid valves, 310 and 320). In addition, a pressure sensor 330 may be provided at a predetermined position to sense pressure applied to the at least one valve 310, 320 or the driving units 130, 131, and control of the driving units 130, 131 may be performed through such sensing.

The compressor 300 and/or the at least one valve 310, 320 may be controlled by the controller (400).

The controller 400 may control the driving units 130, 131 by controlling the compressor 300 and/or the at least one valve 310, 320. For example, the controller 400 may control the driving units 130, 131 to repeatedly advance and retreat the pressing plates 120, 121 at a predetermined frequency. In such a case, apart from increasing the skin area contacting the electrode pair 110 through the pressing plates 120, 121, a massage effect is provided in which fat tissue of the skin is destroyed or softened through physical stimulation of the pressing plates 120, 121, thereby further increasing the fat decomposition effect.

Of course, as described above, the electrode pair 110 may also be implemented to be able to advance and retreat by a driving unit, and in such a case, the controller 400 may control the driving unit to repeatedly advance and retreat the electrode pair 110 at a predetermined frequency. In such a case as well, a massage effect through physical stimulation occurs.

The controller 400 may control the electrode pair 110 according to driving of the pressing plates 120, 121 to differentially perform whether to irradiate electrical energy or the irradiation intensity.

Controlling whether to irradiate electrical energy or the irradiation intensity according to driving of the pressing plates 120, 121 may mean that whether to irradiate electrical energy can be controlled according to the position of the pressing plates 120, 121, such as irradiating electrical energy only when the pressing plates 120, 121 have advanced into the cavity by a predetermined level or more. Further, the controller 400 may adjust the intensity of the electrical energy to be irradiated according to the extent to which the pressing plates 120, 121 advance into the cavity. For example, the intensity of electrical energy irradiated when the pressing plates 120, 121 have advanced to a maximum level and when they have advanced by about 50% may be set differently. Of course, regardless of the degree of advancement, electrical energy of the same intensity may be irradiated when the pressing plates 120, 121 advance by a predetermined level or more.

According to one embodiment, the controller 400 may repeatedly advance and retreat the pressing plates 120, 121 at a predetermined frequency, and in this case, the controller 400 may irradiate electrical energy during advancement and not irradiate electrical energy during retreat, in synchronization with the driving cycle of the pressing plates 120, 121.

According to another embodiment, the controller 400 may not immediately irradiate electrical energy upon advancement of the pressing plates 120, 121, but may irradiate electrical energy after a predetermined time. For example, the controller 400 may repeatedly advance and retreat the pressing plates 120, 121 at a predetermined frequency, and after such repetitive advancement and retreat, once the pressing plates 120, 121 are fixed in an advanced state, the controller 400 may irradiate electrical energy through the electrode pair 110 for a predetermined time.

Those skilled in the art would readily infer that the controller 400 can control driving of the pressing plates 120, 121 and irradiation of electrical energy through the electrode pair 110 in various ways.

For this, the controller 400 may include a predetermined processor, a storage device, and/or a program stored in the storage device and executed by the processor.

The controller 400 may perform computations and functions of controlling other components (for example, the driving units 130, 131, the vacuum pump 200, the compressor 300, various valves, etc.) for implementing the technical concept of the present invention, and may include a processor, a storage device, and a program stored in the storage device and executed by the processor. In this specification, that the controller 400 performs a predetermined operation may be performed by a series of data processing and/or control performed by executing the program by the processor, as would be readily inferred by those skilled in the art.

Meanwhile, a predetermined temperature sensor 600 may be provided at a predetermined position of the skin accommodating cap 100, and thus an increase in skin temperature due to physical stimulation and/or irradiation of electrical energy may be sensed. When it is sensed by the temperature sensor 600 that the skin temperature rises to a predetermined reference temperature, the skin treatment apparatus 1000 may terminate operation.

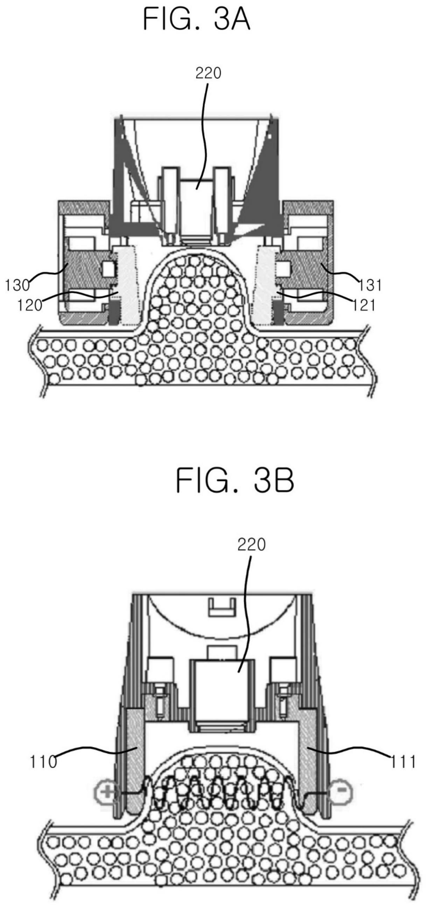

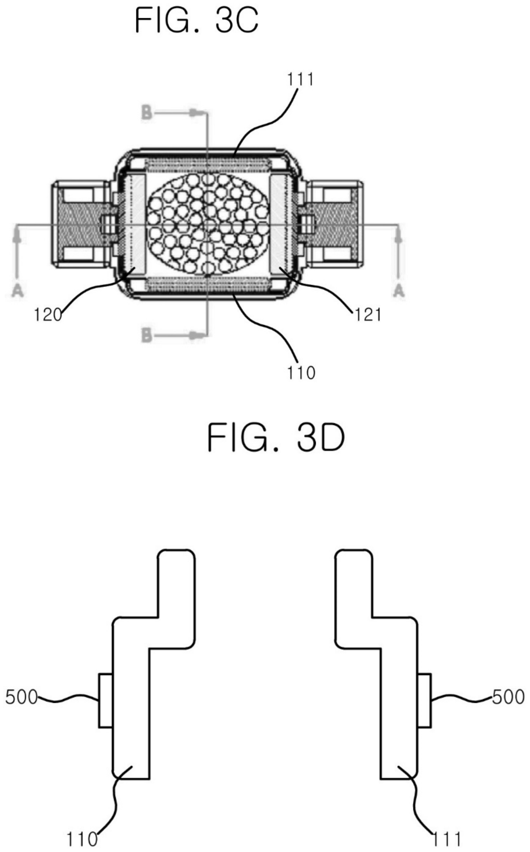

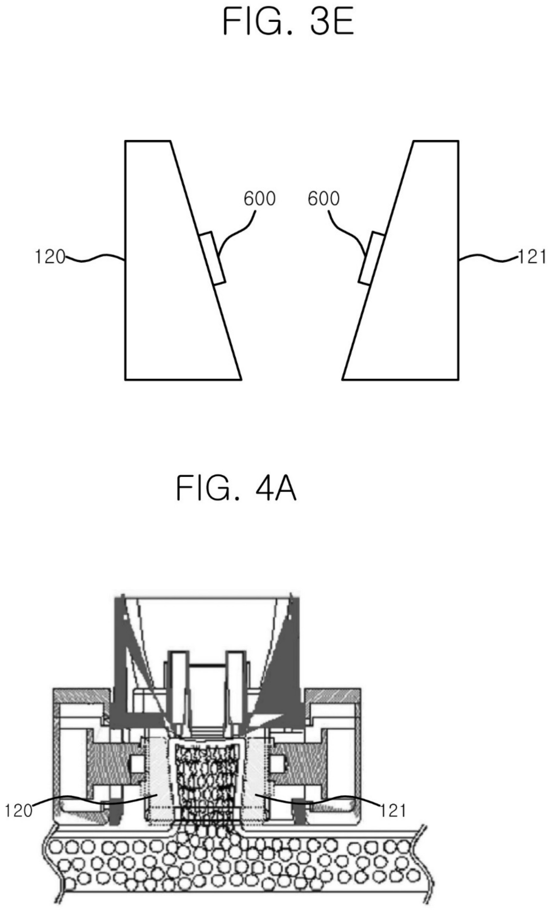

FIG. 3 is a view for explaining a skin accommodating cap of a skin treatment apparatus according to an embodiment of the present invention.

First, FIG. 3a shows a cross-section taken along line A-A of the plan view of the skin accommodating cap 100 shown in FIG. 3c, and FIG. 3b shows a cross-section taken along line B-B of the plan view.

As disclosed in FIGS. 3a to 3c, an electrode pair 110, 111 is disposed to face each other in a first direction (for example, an up-down direction in the plan view) toward the inside of the cavity of the skin accommodating cap 100, and the skin suctioned into the cavity can contact the electrode pair 110, 111.

In addition, pressing plates 120, 121 may be disposed to face each other in a direction different from that of the electrode pair 110, 111 (for example, a left-right direction in the plan view). Such pressing plates 120, 121 can advance into and retreat from the inside of the cavity by the driving units 130, 131.

Meanwhile, as shown in FIGS. 3a and 3e, the pressing plates 120, 121 may be formed to be inclined at a predetermined angle such that a lower portion of an inner surface facing the inside of the cavity protrudes inward more than an upper portion. When negative pressure is applied to the cavity through the through-hole 220, the target portion of the suctioned skin is suctioned into a mountain-peak shape such that a lower width is wide and becomes narrower upward. When the inner surfaces of the pressing plates 120, 121 have such an inclined shape in which the lower portion protrudes more, it may be easier to push the skin portion in the cavity upward compared to a case otherwise, and thus the area contacting the electrode pair 110, 111 can be rapidly increased.

Of course, depending on the embodiment, the inner surfaces of the electrode pair 110, 111 may also be implemented to have an inclined shape in which the lower portion protrudes more.

Meanwhile, as shown in FIG. 3d, a predetermined cooling module 500 may be attached to the outside of the electrode pair 110, 111 to cool heating of the electrode pair 110, 111. The cooling module 500 may be implemented as, for example, a thermoelectric module using a thermoelectric element, but is not limited thereto, and any configuration capable of cooling the electrode pair 110, 111 is sufficient. Through such a cooling module 500, overheating of the electrode pair 110, 111 can be prevented and skin treatment time can be extended.

Also, as shown in FIG. 3e, a predetermined temperature sensor 600 may be attached to the inner surface of the pressing plates 120, 121. In such a case, it may be implemented to directly contact the skin to sense the skin temperature. Of course, as shown in FIG. 2, the temperature sensor 600 may be provided at a predetermined position on an upper portion of the skin accommodating cap 100, and in such a case, it may be implemented as an infrared temperature sensor capable of sensing temperature in a non-contact manner.

Then, the controller 400 may control the driving units 130, 131 or control whether the skin treatment apparatus 1000 is operated based on the temperature sensed by the temperature sensor 600.

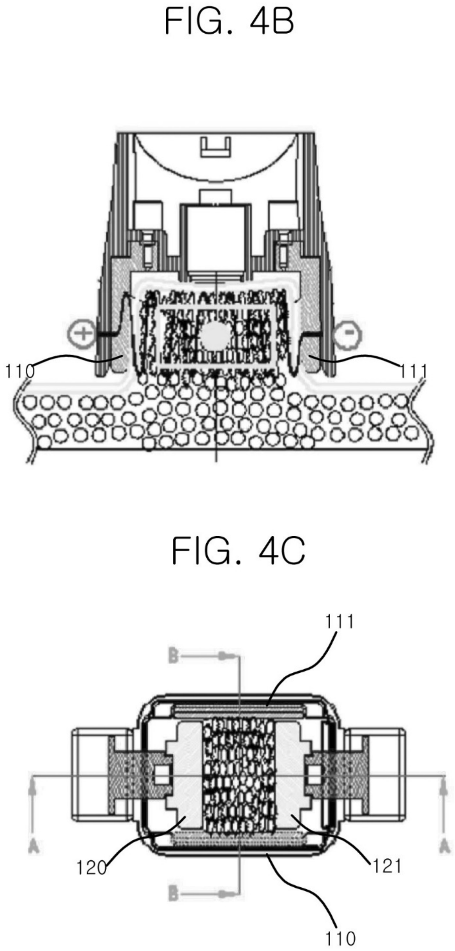

FIG. 4 is a view for explaining the inside of a skin accommodating cap when a pressing plate has advanced according to an embodiment of the present invention.

First, FIG. 4a shows a cross-section taken along line A-A of the plan view of the skin accommodating cap 100 shown in FIG. 4c, and FIG. 4b shows a cross-section taken along line B-B of the plan view.

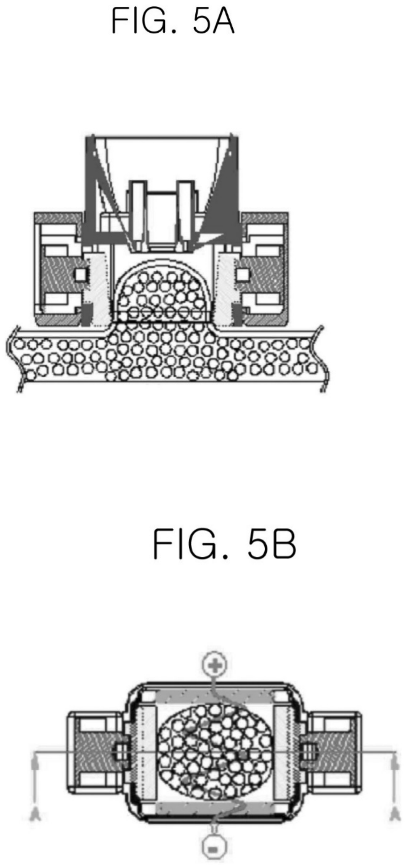

As shown in FIGS. 4a to 4c, when the pressing plates 120, 121 advance into the cavity by predetermined distances, respectively, the skin suctioned into the cavity is pushed from both sides toward the center direction, and in such a case, as shown in FIG. 4b, it can be seen that the suctioned skin contacts the electrode pair 110, 111 over a relatively large area. FIGS. 5 to 7 are views for explaining effects according to a driving method of the skin treatment apparatus according to an embodiment of the present invention.

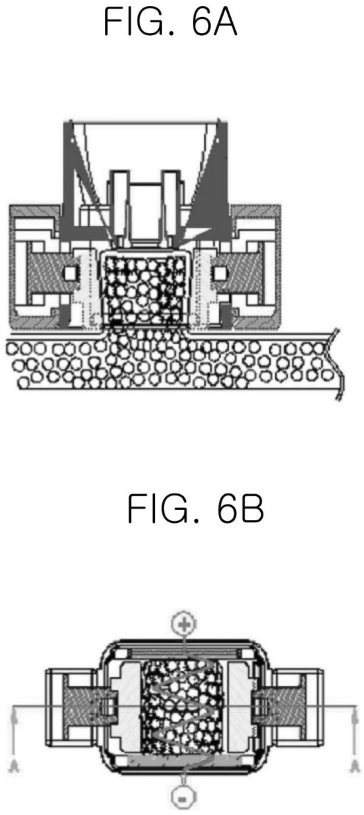

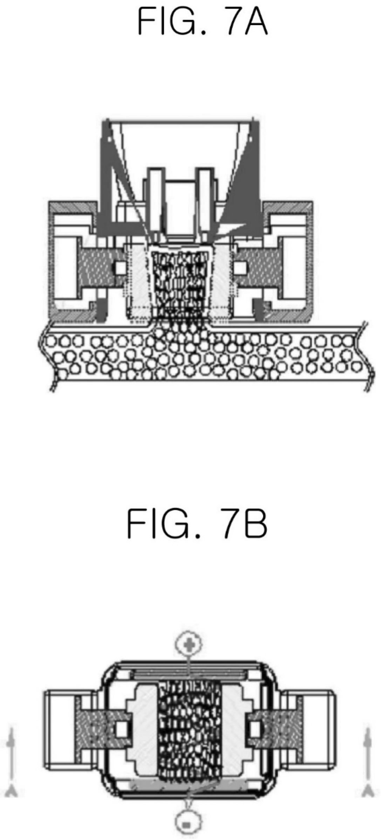

FIGS. 5a and 5b are views illustrating shape changes of the skin in the skin accommodating cap 100 before the pressing plates 120, 121 advance, in different directions; FIGS. 6a and 6b are views illustrating shape changes of the skin in the skin accommodating cap 100 when the pressing plates 120, 121 have advanced only partially (for example, 50% of the maximum), in different directions; and FIGS. 7a and 7b are views illustrating shape changes of the skin in the skin accommodating cap 100 after the pressing plates 120, 121 have advanced to the maximum, in different directions.

First, as shown in FIGS. 5a and 5b, before the pressing plates 120, 121 advance, the area in which the skin suctioned into the cavity contacts the electrode pair 110, 111 is relatively small, and accordingly, when electrical energy is irradiated, the skin region to which electrical energy is irradiated (the region indicated by the red wave in FIG. 5b) is also relatively small.

Then, as shown in FIGS. 6a and 6b, when the pressing plates 120, 121 advance only partially (for example, 50% of the maximum), the area in which the skin suctioned into the cavity contacts the electrode pair 110, 111 becomes relatively larger than that shown in FIG. 5, and accordingly, when electrical energy is irradiated, the skin region to which electrical energy is irradiated (the region indicated by the red wave in FIG. 6b) also becomes relatively larger.

Then, as shown in FIGS. 7a and 7b, when the pressing plates 120, 121 advance to the maximum, the area in which the skin suctioned into the cavity contacts the electrode pair 110, 111 may become maximum. Accordingly, compared to FIG. 5, the area becomes relatively larger, and when electrical energy is irradiated, the skin region to which electrical energy is irradiated (the region indicated by the red wave in FIG. 7b) can be irradiated to almost the entire suctioned skin region. In addition, when the pressing plates 120, 121 advance to the maximum in this way, due to physical pressure applied to the skin, fat molecules inside the skin are destroyed or softened, thereby increasing the efficiency of fat decomposition through electrical energy, and when advancement and retreat of the pressing plates 120, 121 are repeatedly performed at a predetermined frequency, such effects can be further increased.

Although FIGS. 5 to 7 illustrate a case in which the pressing plates 120, 121 are driven by the driving units 130, 131, as described above, the electrode pair 110, 111 may also be driven by a driving unit to advance into the cavity, and even in such a case, effects as described above can be obtained.

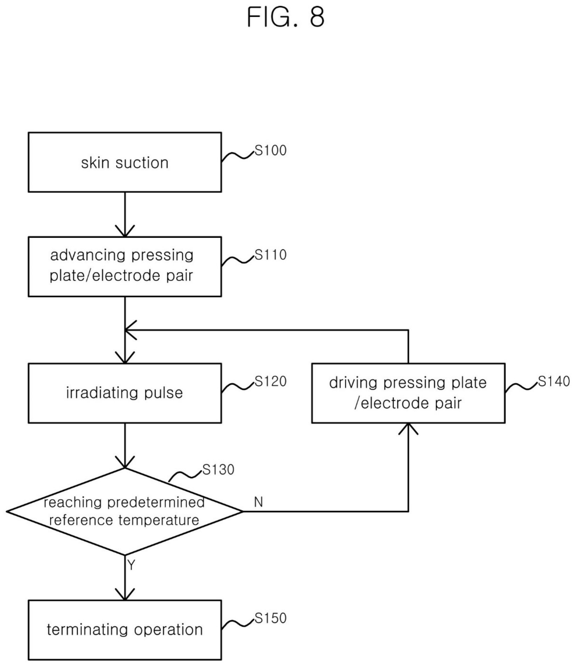

FIG. 8 is a view illustrating a schematic flow chart for explaining a driving method of the skin treatment apparatus according to an embodiment of the present invention.

Referring to FIG. 8, the skin treatment apparatus 1000 may suction a target portion of skin into the cavity of the skin accommodating cap 100(S100). To this end, the skin treatment apparatus 1000 may sense negative pressure applied to the cavity through a predetermined pressure sensor and suction the target portion of skin while increasing the negative pressure until a predetermined reference negative pressure is reached.

Then, the skin treatment apparatus 1000 may advance the pressing plates 120, 121 and/or the electrode pair 110, 111 (S110). Through this, the area of skin contacting the electrode pair 110, 111 can be increased.

Thereafter, the skin treatment apparatus 1000 may irradiate electrical energy (pulses) (S120).

The skin treatment apparatus 1000 may continuously irradiate electrical energy in a fixed state after a one-time advancement of the pressing plates 120, 121 and/or the electrode pair 110, 111, but as described above, physical pressure may also be applied by repeatedly advancing and retreating the pressing plates 120, 121 and/or the electrode pair 110, 111 at a predetermined frequency (S140).

Further, through this process, when the skin temperature in the cavity reaches a predetermined reference temperature (for example, 42°C), the skin treatment apparatus 1000 may stop irradiation of electrical energy and also stop driving of the pressing plates 120, 121 and/or the electrode pair 110, 111, thereby terminating operation of the skin treatment apparatus 1000 (S150).

Accordingly, according to the technical concept of the present invention, in a case of suctioning the skin and irradiating electrical energy, it is possible not only to improve the therapeutic effect by increasing the area of skin contacting the electrodes, but also to further enhance the effect by providing a massage effect through physical external force.

The above description of the present invention is for illustrative purposes, and those skilled in the art will understand that the present invention may be easily modified into other specific forms without changing the technical concept or essential features of the present invention. Therefore, the embodiments described above should be understood as illustrative in all respects and not restrictive. For example, each component described as a single unit may be implemented in a distributed manner, and likewise, components described as distributed may be implemented in a combined form. The scope of the present invention is defined by the appended claims rather than the detailed description, and all changes or modifications derived from the meaning and scope of the claims and their equivalent concepts should be construed as being included in the scope of the present invention.

### [INDUSTRIAL APPLICABILITY]

The present invention may be used for a skin treatment apparatus and a driving method thereof.

## Claims

1. A skin treatment apparatus comprising:
a skin accommodating cap having an open lower surface and a cavity therein;
an electrode pair provided such that, when a target portion of skin is suctioned toward the cavity of the skin accommodating cap, the suctioned skin comes into contact within the cavity of the skin accommodating cap, the electrode pair being disposed to face each other in a first direction;
a driving unit configured to drive a pressing plate so that the pressing plate can advance into and retreat from an inner direction of the cavity;
a controller for controlling the driving unit; and
at least one pressing plate which is driven by the driving unit so as to be able to advance into the cavity from a second direction different from the first direction and which increases an area of contact with the electrode pair by pressing inward the skin suctioned into the cavity.

2. The skin treatment apparatus of claim 1, wherein the skin treatment apparatus comprises a pair of pressing plates disposed to face each other in the second direction.

3. The skin treatment apparatus of claim 1, wherein the controller controls the electrode pair to differentially perform whether to irradiate an RF pulse or an irradiation intensity according to driving of the pressing plate.

4. The skin treatment apparatus of claim 1, wherein at least one of the at least one pressing plate or the electrode pair is formed to be inclined at a predetermined angle such that a lower portion of an inner surface facing the inside of the cavity protrudes inward more than an upper portion thereof.

5. The skin treatment apparatus of claim 1, wherein the driving unit further drives the electrode pair so that the electrode pair can advance into and retreat from the inner direction of the cavity.

6. The skin treatment apparatus of claim 1, further comprising a cooling module capable of cooling the electrode pair.

7. The skin treatment apparatus of claim 1, further comprising a vacuum pump that provides negative pressure so that the target portion of the skin can be suctioned toward the cavity of the skin accommodating cap.

8. The skin treatment apparatus of claim 1, wherein the driving unit repeatedly advances and retreats at least one of the pressing plate or the electrode pair at a predetermined frequency.

9. The skin treatment apparatus of claim 1, further comprising a temperature sensor for sensing a temperature of the skin suctioned into the cavity, wherein the controller controls the driving unit or controls operation of the skin treatment apparatus based on the sensed temperature.

10. A skin treatment apparatus comprising:
a skin accommodating cap having an open lower surface and a cavity therein;
an electrode pair provided such that, when a target portion of skin is suctioned toward the cavity of the skin accommodating cap, the suctioned skin comes into contact within the cavity of the skin accommodating cap, the electrode pair being disposed to face each other in a first direction;
a driving unit configured to drive the electrode pair so that the electrode pair can advance into and retreat from an inner direction of the cavity; and
a controller for controlling the driving unit,
wherein the electrode pair is driven by the driving unit to press inward the skin suctioned into the cavity, thereby increasing an area of contact with the electrode pair.

11. A driving method of a skin treatment apparatus, the method comprising:
suctioning a target portion of skin toward a cavity of a skin accommodating cap by the skin treatment apparatus;
advancing a pressing plate into the cavity from a second direction different from a first direction of an electrode pair disposed in the first direction so that the suctioned skin comes into contact with the electrode pair within the cavity of the skin accommodating cap; and irradiating electrical energy through the electrode pair by the skin treatment apparatus.

12. The driving method of claim 11, further comprising repeatedly advancing and retreating at least one of the pressing plate or the electrode pair at a predetermined frequency by the skin treatment apparatus.

13. A driving method of a skin treatment apparatus, the method comprising:
suctioning a target portion of skin toward a cavity of a skin accommodating cap by the skin treatment apparatus;
advancing an electrode pair disposed in a first direction into an inner side of the cavity so that the suctioned skin comes into contact with the electrode pair within the cavity of the skin accommodating cap; and
irradiating electrical energy through the electrode pair by the skin treatment apparatus.
